# EUROPEAN PATENT APPLICATION

(11) **EP 1 382 349 A1**
(43) Date of publication of application: **21.01.2004**
(21) Application number: 02720595.4
(22) Date of filing: 25.04.2002
(51) Int. Cl.: A61K 45/00, A61K 31/198, A61P 43/00, A61P 35/00

(54) **SUPPLEMENTARY IMMUNOTHERAPEUTICS TO BE USED AFTER LUNG CANCER REMOVAL**

(30) Priority: 27.04.2001 JP 2001133687; 27.04.2001 JP 2001133700
(71) Applicant: Nippon Kayaku Kabushiki Kaisha, Tokyo 102-8172 (JP)
(72) Inventor: OGINO, Teruo, Kawagoe-shi, Saitama 350-1108 (JP); SAWADA, Hitoshi, Misato-shi, Saitama 341-0013 (JP); ABE, Fuminori, Kita-ku, Tokyo 115-0042 (JP); ONOSE, Junko, Bunkyo-ku, Tokyo 113-0024 (JP); YAMASAKI, Emiko, Mitaka-shi, Tokyo 181-0001 (JP); TAKEMASA, Hiroaki, Kita-ku, Tokyo 115-0042 (JP)
(74) Representative: Gille Hrabal Struck Neidlein Prop Roos
(86) International application number: PCT/JP2002/004126
(87) International publication number: WO 2002/087617

(57) **Abstract**

The present invention relates to an agent for prolonging recurrence-free survival time of patients with lung squamous cell carcinoma in the histopathological stage I or an agent for prolonging the survival time or for an agent for prolonging survival time, particularly recurrence-free survival time of patients after surgical resection of lung squamous cell carcinoma in the histopathological stage IB, containing an aminopeptidase inhibitor such as ubenimex as the active ingredient.

## Description

### Technical Field

The present invention relates to an agent for prolonging the survival time, particularly recurrence-free survival time of patients after resection of lung squamous cell carcinoma in the histopathological stage I, particularly the stage IB, containing an aminopeptidase inhibitor as the active ingredient and serving as an adjuvant immunotherapeutic agent for such patients.

### Background Art

As the standard therapeutics of non-small cell lung carcinoma typically including lung squamous cell carcinoma at the pathological stages I and II, surgical resection (resection of lung lobes + absolute eradicative resection of mediastinal lymph nodes) is selected. However the recurrence ratio of lung carcinoma is relatively high and a great number of postsurgery adjuvant therapeutics by radiotherapy, chemotherapy and immunotherapy have been attempted and reported, so as to prolong the survival time and/or recurrence-free survival time. Although the postsurgery adjuvant radiotherapy may decrease the frequency of topical recurrence after surgery, the said radiotherapy has not yet made any fruitful result of the increase in survival ratio, partially because postsurgery recurrence frequently occurs as remote hematogenous metastasis rather than local metastasis (Van Houtte P et al: Int. J. Radiat. Oncol. Biol. Phys . , 6, 983-986 (1980); Weisenburger TH: N. Engl. J. Med., 315, 1377-1381 (1986)). The postsurgery adjuvant chemotherapy has not yet brought about a sufficient effect due to poor compliance derived from adverse effects of chemotherapeutic agents ( Feld R et al : J . Natl . Cancer Inst., 85, 299-306 (1993)). Nonetheless, a significant increase of the survival ratio by the long-term oral administration of UFT ( tegaful + uracil) ( Wada H et al : J . Clin . Oncol., 14, 1048-1054 (1996)) and the improvement of the survival ratio by cisplatin administration (Non-small Cell Lung Cancer Collaborative Group: BMJ. , 311, 899-909 (1996)) have been reported in recent years. In the postsurgery adjuvant immunotherapeutics, the therapy for modifying the response potency responsible for cancer development and progression in a host against cancer cells is defined as biological response modifier (BRM). Since it was elucidated in the 1980's for cytokine to work as a regulatory factor in specific and non-specific immune responses to cancer, research works using cytokine has made a progress. Interferon-α, interferon-β, interferon-γ, interleukin-2, granulocyte colony stimulation factor (G-CSF) and the like have already clinically applied. Lately, the elucidation of T helper (Th) cell subsets has also made a progress. It is reported that Th1 cell is important for the induction of cell-damaging T cells with anti-cancer actions and that Th1 cells generate interleukin-2, interferon-γ, and interleukin-12 ( Recent Advances of Human Tumor Immunology and Immunotherapy, Japan Scientific Societies Press, Tokyo, 1999).

The active ingredient ubenimex [chemical name: (-)-N-((2S, 3R)-3-amino-2-hydroxy-4-phenylbutyryl)-L-leucine] to be used in the invention is applied clinically as an anti-cancer antibiotic concurrently with a chemotherapeutic agent for keeping and enforcing the management and control of adult acute non-lymphocytic leukemia after complete remission is introduced. The action mechanisms reported so far include the competitive inhibitory action against leucine aminopeptidase by inhibiting the aminopeptidase on cell membrane surface (JP-B-54-39477), the suppressive action of cancer proliferation, the action to release interleukin-1 and -2, the anti-fungal action (JP-A-5-117146) and the cervical canal-ripening action (JP-A-8-67629). It is also reported in regards to the safety profile of ubenimex that ubenimex is a compound with no observed toxicity in mice when a dose of 298 mg/kg (i.p.) was administered (J. Antibiotics, 29 , 97-99 (1976)) and with no specific adverse effects observed in humans when a dose of 10 to 900 mg/kg (i.p.) was administered (Saito K et al.: Jpn. J. Cancer Chemother., 10 . 211-217 (1983); Yugeta E et al: Recent Results of Bestatin, Tokyo, 101-112 (1986)).

Yasumitu T et al . report about a clinical trial of ubenimex in resection cases of lung squamous cell carcinoma that in groups of the cases with concurrent administration of tegaful and with no concurrent tegaful administration (21 cases: administration of 30 mg ubenimex daily for one year or longer) , the survival time of the ubenimex-administered group in the histopathological stage I was prolonged, compared with the ubenimex non-administered group at the stage (Yasumitu T et al.: Acta. Oncol., 29, 827-831 (1990)). However, because the number of these cases at the clinical trial is small, it is described that additional examinations would be required. There is a report (C. Mouritzen: Acta. Oncol. , 29, 817-820 (1990) that resection cases of non-small cell lung cancer, mainly squamous cell carcinoma and lung adenocarcinoma cases, it seems that from the result of the case studies of the administration of ubenimex alone in a relatively large number of cases in the histopathological stages I and II (90 mg twice weekly for 5 years in 227 cases) the ubenimex-administered group has favorable trend compared with the non-administered group in terms of recurrence-free survival time but the trend is far from significance. As described above, the prolongation of the survival time by ubenimex of patients with resection of lung squamous cell carcinoma at the stage I is not definite owing to a small number of the cases despite the presence of the report of Yasumitu et al. In view of the prolongation of recurrence-free survival time alone, the report of C. Mouritzen with a relatively great number of the cases in the stages I and I I is, on the contrary, negative about the effect of ubenimex stating"the effect is far from significance". Further, if the case is limited to the patients with lung squamous cell carcinoma at the stage IB, particularly such patients after surgical resection of the lung squamous cell carcinoma, no report about a adjuvant immunotherapeutic agent with an action prolonging the survival time of such patients , particularly the recurrence-free survival time thereof has been issued yet.

### Disclosure of the Invention

The present inventors have closely investigated into the possibility for adjuvant immunotherapeutic agents of aminopeptidase inhibitors such as ubenimex as in a great number of cases. Consequently, the inventors have found that aminopeptidase inhibitors can significantly improve the survival time, particularly 5-year recurrence-free survival ratio of cases after complete resection of lung squamous cell carcinoma in the histopathological stage I (total 389 cases) with no deterioration of the QOL of these patients and that the separate analysis of patients at the stage IA and patients at the stage IB clearly shows that the survival ratio and recurrence-free survival ratio of the patients at the stage IB are far more improved, compared with those of the patients at the stage IA. Thus, the inventors have achieved the invention. The aforementioned reports of ubenimex never include any descriptionsuggestingsuchfindings, whichhavebeenfoundfirst by the inventors. Further, the invention has been accomplished by tests with a larger number of enrolled patients as to have sufficient reliability on the statistic analysis.

In other words, the invention relates to the following (1) to (17).
(1) An adjuvant immunotherapeutic agent containing an aminopeptidase inhibitor as the active ingredient for use after surgical resection of lung squamous cell carcinoma in the histopathological stage IB.
(2) The adjuvant immunotherapeutic agent according to (1), where the aminopeptidase inhibitor is ubenimex or a pharmacologically acceptable salt thereof.
(3) The adjuvant immunotherapeutic agent according to (1) or (2), which is used for the prolongation of the survival time or recurrence-free survival time of patients after surgical resection of lung squamous cell carcinoma in the histopathological stage IB.
(4) The adjuvant immunotherapeutic agent d according to (2), which is ubenimex or one kind of pharmacologically acceptable salt thereof to be orally administered at 5 to 500 mg/adult · day to patients after surgical resection of lung squamous cell carcinoma in the histopathological stage IB.
(5) The adjuvant immunotherapeutic agent according to (4), which is used for ubenimex or one kind of pharmacologically acceptable salt thereof to be orally administered daily at 10 to 300 mg/adult • day for at least two years after the surgical resection.
(6) The adjuvant immunotherapeutic agent according to (1) or (4), where the surgical resection of lung squamous cell carcinoma is complete surgical resection of lung squamous cell carcinoma.
(7) An agent for prolonging the survival time after complete surgical resection of lung squamous cell carcinoma in the histopathological stage IB, containing ubenimex or a pharmacologically acceptable salt thereof as the active ingredient.
(8) The agent for prolonging the recurrence-free survival time after complete surgical resection of lung squamous cell carcinoma in the histopathological stage IB, containing ubenimex or a pharmacologically acceptable salt thereof as the active ingredient.
(9) The method for prolonging the survival time of patients after complete surgical resection of lung squamous cell carcinoma in the histopathological stage IB, characterized by administering an effective dose of ubenimex or a pharmacologically acceptable salt thereof to the patients.
(10) Use of ubenimex or a pharmacologically acceptable salt thereof for prolonging the survival time of patients after complete surgical resection of lung squamous cell carcinoma in the histopathological stage IB.
(11) An agent for prolonging the survival time with no recurrence of lung squamous cell carcinoma in the histopathological stage I, the agent containing an aminopeptidase inhibitor as the active ingredient.
(12) The agent for prolonging the survival time with no recurrence of lung squamous cell carcinoma according to (11), where the aminopeptidase inhibitor is ubenimex or a pharmacologically acceptable salt thereof.
(13) The agent for prolonging the survival time with no recurrence of lung squamous cell carcinoma according to (11) or (12), which is administered after surgical resection of the carcinoma.
(14) An agent for prolonging the survival time with no recurrence of lung squamous cell carcinoma according to (13), where the surgical resection is complete surgical resection.
(15) The agent for prolonging the survival time with no recurrence of lung squamous cell carcinoma according to (13), which is orally administered daily for at least two years after the surgery.
(16) The agent for prolonging the recurrence-free survival time of patients in the histopathological stage I of lung squamous cell, the agent containing ubenimex or a pharmacologically acceptable salt thereof as the active ingredient for oral administration at 5 to 500 mg/adult • day on the active ingredient basis after complete surgical resection of lung squamous cell carcinoma in the histopathological stage I.
(17) The agent for prolonging the survival time with no recurrence of lung squamous cell carcinoma described in the aspect (16), wherein ubenimex or a pharmacologically acceptable salt thereof is orally administered daily at 10 to 300 mg/adult • day for at least two years after the surgery.

### Brief Description of the Drawings

Fig.1 shows graphs depicting the recurrence-free survival time curves of an ubenimex group and a placebo group for cases in the histopathological stage I.
Fig.2 shows graphs depicting the survival time curves of an ubenimex group and a placebo group for cases in the histopathological stage I.
Fig.3 shows graphs depicting the survival time curves of an ubenimex group and a placebo group for cases in the histopathological stage IB.
Fig.4 shows graphs depicting the survival time curves of an ubenimex group and a placebo group for cases in the histopathological stage IA.
Fig. 5 shows graphs depicting the recurrence-free survival time curves of an ubenimex group and a placebo group for cases in the histopathological stage IB.
Fig. 6 shows graphsdepicting the recurrence-free survival time curves of an ubenimex group and a placebo group for cases in the histopathological stage IA.

### Description of symbols: "U" in Figs. 1 to 6 means ubenimex group; and "P" means placebo group.

### Best Mode for Carrying out the Invention

In accordance with the invention, aminopeptidase inhibitors are used as the active ingredient. Aminopeptidase is an enzyme catalyzing the breakage of bonds near the amino terminus of polypeptides specifically existing on the membrane surface of immune cells. It is reported that aminopeptidase plays important roles in cell proliferation and differentiation and signal transmission via peptides. Aminopeptidase inhibitors are substances inhibiting the enzyme activities, including for example ubenimex, actinonine ( chemical name; (3-(1- [2-(hydroxymethyl)-1-pyrrolidinyl] carbonyl) - 2-methylpropyl)carbamoyl) octanohydroxamic acid), amastatin [ chemical name; {(2S, 3S)-3-amino-2-hydroxy-5-methylhexanoyl}-valine-valine-aspar agine ] , arphamenine A ( chemical name; (2R, 5S)-5-amino-8-guanid-4-oxo-2-phenylmethyloctanoic acid] , arphamenine B [ chemical name; (2R, 5S)-5-amino-8-guanid-4-oxo-2-p-hydroxyphenylmethyloctanoic acid ] , probestin [ chemical name; (2S, 3R)-3-amino-2-hydroxy-4-phenylbutanoyl-leucine-leucine-prol ine ] , leuhistin [ chemical name; (2R, 3S)-3-amino-2-hydroxy-2-(1H-imidazol-4-ylmethyl)-5-methylhe xanoic acid ] , and febestatin [ chemical name; [ (2S, 3R)-3-amino-2-hydroxy-4-phenylbutanoyl-valine-phenylalanine ] . Ubenimex is preferable. Ubenimex is used in the free form, or pharmacologically acceptable salt forms thereof with acids or bases. The salts with acids include for example the salts with hydrochloric acid, sulfuric acid and phosphoric acid, while the salts with bases include for example the salts with alkali metals such as sodium and potassium.

In accordance with the invention, the histopathological stage I means in case of for example lung cancer, the stages of lung cancer including both lung cancer in the histopathological stage IA (T1N0M0) and the histopathological stage IB (T2N0M0) according to the TNM classification (1997). According to the TNM classification, thehistopathological stage IA means the stage that the maximum diameter of the tumor is 3 cm or less, that the tumor is enclosed with lung tissues or visceral pleura and that never reach the infiltration of the tumor into the part central from the bronchi lobares under bronchoscope observation (in other words, the cancer has not yet inf iltrated into the main airway) (T1) and that never involves metastasis at lymph nodes or remote metastasis (N0M0). According to the TNM classification, additionally, the histopathological stage IB means the stage of cancer with one of the following tumor size and progression stages (T2):
<1> the maximum diameter of cancer above 3 cm;
<2> cancer having been infiltrated into a main bronchial, of which the center is nevertheless 2 cm or more apart from thebifurcatio tracheae;
<3> cancer having been infiltrated into visceral pleura;
<4> cancer involving atelectasis or obstructive pneumonia reaching the hilum of lung but never reaching unilateral lung, and with no metastasis in lymph node or no remote metastasis (N0M0).

In accordance with the invention, the term recurrence-free survival time means the period in which a patient can survive with no recurrence of carcinoma. The term "recurrence of carcinoma" represents a case diagnosed again with carcinoma after carcinoma therapy such as surgical resection of carcinoma and a case in which carcinoma cells are detected again after the confirmation of disappearance of carcinoma cells. Herein, recurrent carcinoma is not limited to for example lung carcinoma but includes various malignant tumor such as brain cancer.

In accordance with the invention, the term "adjuvant immunotherapeutic agent" means a pharmaceutical agent with an action of enhancing the immune response potency of immunocompetent cells of a host after lung cancer therapy, to enhance the anti-carcinoma effect.

The term "agent for prolonging survival time" means a pharmaceutical agent having an action to prolong survival time. The survival time means the period in which a patient survives in the presence or absence of the recurrence of carcinoma.

In accordance with the invention, the term "agent for prolonging recurrence-free survival time" means a pharmaceutical agent having an action to prolong the survival time without recurrence of carcinoma.

The pharmaceutical agent of the invention, namely the adjuvant immunotherapeutic agent can be administered in oral dosage forms for example tablets, capsules, powders, granules, liquids, and dry syrups, or in parenteral dosage forms for example injections and suppositories to target patients in accordance with the invention, namely patients with lung squamous cell carcinoma in the histopathological stage I, preferably at the stage IB. The patients are preferably such patients after carcinoma therapy, more preferably such patients after surgical resection of the carcinoma. The most appropriate patients are patients with lung squamous cell carcinoma in the histopathological stage I, preferably at the stage IB and after complete surgical resection of the carcinoma. Herein, complete resection represents the state of carcinoma absolutely resected grossly or pathologically.

The dose is appropriately determined, depending on a kind of an aminopeptidase inhibitor and the age, body weight and symptoms of a patient and the therapeutic objective. An effective dose of each drug is administered. When ubenimex or a salt thereof is used as the aminopeptidase inhibitor, generally, the effective dose of ubenimex for parenteral administration is about 1 to 300 mg/adult•day and the effective dose thereof for oral administration is 5 to 500 mg/adult • day, preferably 10 to 300 mg/adult • day, more preferably 10 to 200 mg/adult • day. The dose of another aminopeptidase inhibitor should be determined similarly in the case of ubenimex, taking into account the strength of the inhibitory activity of the aminopeptidase inhibitor and the toxicity thereof. Preferably, ubenimex or a salt thereof is orally administered. Generally, ubenimex or a salt thereof is preferably administered for at least two years daily (or in a similar administration) after surgery. As to the longest dosing period, additionally, ubenimex or a salt thereof can be administered as long as possible, because of almost no occurrence of adverse effects. Generally, ubenimex or a salt thereof is administered for about 5 years postsurgery. Further, the number of administration per day is not specifically limited. The effective dose is administered once daily.

The individual pharmaceutical agents of the invention mentioned above may be aminopeptidase inhibitors alone or in mixture with excipients or carriers (simply referred to carriers including the both hereinbelow). When aminopeptidase inhibitors are used alone, it may be used as they are. For use in mixture, aminopeptidase inhibitors are mixed with for example carriers and, if necessary, formulated into preparations such as injections, oral preparation or suppositories for use through processes such as charging, molding and drying. As the carriers, pharmaceutically acceptable carriers may be selected, while the kind and composition thereof can be determined appropriately, depending on the administration route and the administration method.

As the carriers, liquid carriers for example water, alcohol, animal and plant oils such as soybean soil, peanut oil, gum oil, and mineral oils, or synthetic oils are used. As solid carriers, sugars such as maltose and sucrose, amino acids, cellulose derivatives such as hydroxypropyl cellulose, starches such as potato starch, organic acid salts such as magnesium stearate are used.

For injections, generally, solvents are used as the liquid carriers and include for example physiological saline, various buffer solutions , aqueous solutions of sugars , aqueous solutions of glycols such as ethylene glycol and polyethylene glycol. The sugars for use in aqueous solutions of sugars include for example glucose, inositol, and mannitol. The carriers for use in freeze-dried preparation for injections include for example sugars such as inositol, mannitol, glucose, mannose, maltose and sucrose, and amino acids such as phenylalanine. In use, the freeze-dried preparation are dissolved in appropriate solvents for injections, for example liquids for intravenous injections, such as sterile water, physiological saline, aqueous glucose solution, aqueous electrolyte solution, and aqueous amino acid solution.

For oral administration, aminopeptidase inhibitors are formulated together with above-mentioned solid carriers or the liquid carriers into preparations such as tablets, capsules, powders, granules, liquids and dry syrups and administered.

The content of an aminopeptidase inhibitor for example ubenimex or a salt thereof in the pharmaceutical agent of the invention varies, depending on the dosage form. Generally, the content is 0.01 to 100 % by weight, preferably 0.02 to 90 % by weight. In case of capsules, tablets, granules and powders, generally, the content of ubenimex or a salt thereof is about 0 . 02 to 90 % by weight, preferably 0.3 to 20 % by weight, and the remaining part is occupied by the carriers.

### Examples

The invention is now specifically described in the following formulation examples and clinical test examples. But the invention is not limited to these examples.

### Formulation Example 1 (Capsule)

| | |
|---|---|
| Ubenimex | 30 parts by weight |
| Potato starch | 77 parts by weight |
| Crystalline lactose | 100 parts by weight |
| Magnesium stearate | 3 parts by weight |

The above ingredients were mixed together; 210 mg of the resulting mixture (containing 30 mg ubenimex) was charged in No.3 capsule to prepare a capsule preparation.

### Formulation Example 2 (Tablet)

Ubenimex (30 parts by weight), crystalline lactose (120 parts by weight), crystalline cellulose (147 parts by weight) and magnesium stearate (3 parts by weight) were mixed together in a mixer of the V type, followed by tableting, to prepare a tablet of 300 mg (containing 30 mg ubenimex).

### Clinical test Example

With complete resection patients of lung squamous cell carcinoma in the histopathological stage I, a multicenter double blind comparative test using an ubenimex preparation (containing 30 mg ubenimex) and a placebo preparation (a control contentaining no ubenimex) was carried out.

The ubenimex preparation or the placebo preparation was administered orally once a day. The dosing period of these preparations was 2 years after surgical resection. The observation period was 5 years or more including the subsequent period of 3 years or more of no administration after the dosing period. The survival time and recurrence-free survival time in the collected 389 cases (189 cases at the stage IA and 200 cases at the stage IB) were compared between the two groups, namely the ubenimex group and the placebo group by the Kaplan-Meier method.

Tables 1 and 2 show the results of the statistic analysis by the generalized Wilcoxon test and the Log-Rank test. Table 1 shows the results of the statistic analysis of the total cases of the stage I, while Table 2 shows the results of the statistic analysis of the cases grouped in the stage IA and the stage IB. In these tables, the "U group" means the ubenimex-administered group and the "P group" means the placebo-administered group.

Additionally, Figs. 1, 5 and 6 show the recurrence-free survival ratio curves of the ubenimex group and the placebo group, while Figs.2, 3 and 4 show the survival ratio curves thereof. Figs.1 and 2 show the results of the total cases at the stage I ; Figs . 3 and 5 show the results of the cases at the stage IB, while Figs . 4 and 6 show the results of the cases at the stage IA.

**Table 1**

| Analytical items | Number of cases | | | 5-year survival ratio | | Test results | |
|---|---|---|---|---|---|---|---|
| | total | U group | P group | U group | P group | G. Wilcoxon | Log-Rank |
| Survival time | 389 | 199 | 190 | 81 % | 75 % | 0.0572 | 0.0594 |
| Recurrence-free survival time | 389 | 199 | 190 | 70 % | 62 % | 0.0425 | 0.0344 |

As apparently shown in Table 1, the results of the analysis of the total cases in the stage I show that both the survival time and recurrence-free survival time of the ubenimex-administered group are prolonged compared with the non-administered group, and especially that the effect on the prolongation of the recurrence-free survival time is great. Specifically, the data of the recurrence-free survival time show that the 5-year recurrence-free survival ratio in the ubenimex group is 70 %, while in the placebo group, the ratio is 62 %. As the generalized Wilcoxon test value is 0.0425 and the Log-Rank test value is 0.0344, the ubenimex group is shown at a 5 % or lower level of risk rate by both the tests, indicating clearly that ubenimex has an action to prolong the recurrence-free survival time. Meanwhile, the data of the survival time show that the 5-year survival time is 81 % in the ubenimex group while in the placebo group, it is 75 %. The generalized Wilcoxon test value is 0.0572 and the Log-Rank test value is 0.0594. A significant trend was confirmed in the ubenimex group. Herein, the survival time means the period in which a patient can survive regardless of the presence or absence of the recurrence of carcinoma.

**Table 2**

| Analytical items | Pathological stages | Cases | | | 5-year survival ratio | | Test results | |
|---|---|---|---|---|---|---|---|---|
| | | Total | U group | P group | U group | P group | G.Wilcoxon | Log-Rank |
| Survival time | Stage IA | 189 | 96 | 93 | 84 % | 80 % | 0.3708 | 0.3525 |
| | Stage IB | 200 | 103 | 97 | 77 % | 71 % | 0.0726 | 0.0782 |
| Recurrence-free survival time | Stage IA | 189 | 96 | 93 | 73 % | 67 % | 0.2690 | 0.2090 |
| | Stage IB | 200 | 103 | 97 | 68 % | 59 % | 0.0612 | 0.0644 |

As apparently shown in Table 2, the results of the analysis of the total cases at the stage I further divided into IA and IB show an increasing trend in the 5-year survival ratio and 5-year recurrence-free survival ratio of the ubenimex group in the stage IA, compared with the placebo group. However, the p values in the results by any of the generalized Wilcoxon test and the Log-Rank test for the survival time and the recurrence-free survival time are 0.3 or more and 0.2 or more, respectively, which are both far apart from the level of significance, namely 5 %. In case of the stage IB, on the other hand, the 5-year survival ratio and the 5-year recurrence-free survival ratio were distinctively increased in the ubenimex group at a distinct significance tendency was shown, compared with the placebo group. That is concerning an effect on the prolongation of survival time, the p value of the generalized Wilcoxon test was 0.0726, and the p value of the Log-Rank test was at the p value of 0.0782, and concerning the effect on the prolongation of recurrence-free survival time, the p value of the generalized Wilcoxon test was 0.0612 and the p value of the Log-Rank test was 0.0644. The effect on the prolongation of survival time and the effect on the prolongation of survival time are fairly closer to the 5% level of significance, indicating that these effects are great. The test values for the effect on the prolongation of recurrence-free survival time are closer to the 5% level of significance than those for the effect on the prolongation of survival time, indicating that the former effect is greater.

Concerning adverse effects, further, only one adverse effect significantly occurring via ubenimex administration was appetite loss (at an incidence of 2.5 %).

These effects can be attained by administration of ubenimex alone; with no concurrent use of other anti-cancer agents.

These data indicate that ubenimex has a significant action on the prolongation of the survival time, particularly recurrence-free survival time of patients after complete resection of lung squamous cell carcinoma in the histopathological stage I and that the analysis of the cases at the stage I divided into the stages IA and IB shows larger effects in the stage IB than the effects in the stage IA, particularly a larger effect on the prolongation of the recurrence-free survival time.

### Industrial Applicability

In accordance with the invention, the administration of an aminopeptidase inhibitor, particularly a preparation of ubenimex for at least two years after complete surgical resection of lung squamous cell carcinoma can improve the 5-year recurrence-free survival ratio of patients in the histopathological stage I and the recurrence-free survival time thereof can be prolonged with significant difference, without almost adverse effects. Thus, the invention is useful as an agent for prolonging the recurrence-free survival time for postsurgery adjuvant therapy with no QOL deterioration.

## Claims

1. An adjuvant immunotherapeutic agent containing an aminopeptidase inhibitor as the active ingredient for use after surgical resection of lung squamous cell carcinoma in the histopathological stage IB.

2. The adjuvant immunotherapeutic agent according to claim 1, where the aminopeptidase inhibitor is ubenimex [chemical name:(-)-N-((2S, 3R)-3-amino-2-hydroxy-4-phenylbutyryl)-L-leucine ] or a pharmacologically acceptable salt thereof.

3. The adjuvant immunotherapeutic agent according to claim 1 or 2 , which is used for the prolongation of the survival time or recurrence-free survival time after surgical resection of lung squamous cell carcinoma in the histopathological stage IB.

4. The adjuvant immunotherapeutic agent according to claim 2 , which is ubenimex or one kind of pharmacologically acceptable salt thereof to be orally administered at 5 to 500 mg/adult · day after surgical resection of lung squamous cell carcinoma in the histopathological stage IB.

5. The adjuvant immunotherapeutic agent according to claim 4, which is ubenimex or one kind of pharmacologically acceptable salt thereof to be orally administered daily at 10 to 300 mg/adult • day for at least two years after the surgical resection.

6. The adjuvant immunotherapeutic agent according to claim 1 or 4, where the surgical resection of lung squamous cell carcinoma is complete surgical resection of lung squamous cell carcinoma.

7. An agent for prolonging the survival time after complete surgical resection of lung squamous cell carcinoma in the histopathological stage IB, containing ubenimex or a pharmacologically acceptable salt thereof as the active ingredient.

8. An agent for prolonging the recurrence-free survival time after complete surgical resection of lung squamous cell carcinoma in the histopathological stage IB, containing ubenimex or a pharmacologically acceptable salt thereof as the active ingredient.

9. Amethod for prolonging the survival time of patients after complete surgical resection of lung squamous cell carcinoma in the histopathological stage IB, **characterized by** administering an effective dose of ubenimex or a pharmacologically acceptable salt thereof to the patients.

10. Use of ubenimex or a pharmacologically acceptable salt thereof for prolonging the survival time of patients after complete surgical resection of lung squamous cell carcinoma in the histopathological stage IB.

11. An agent for prolonging the recurrence-free survival time of patients with lung squamous cell carcinoma in the histopathological stage I, containing an aminopeptidase inhibitor as the active ingredient.

12. The agent for prolonging the recurrence-free survival time according to claim 11, where the aminopeptidase inhibitor is ubenimex or a pharmacologically acceptable salt thereof.

13. An agent for prolonging the recurrence-free survival time according to claim 11 or 12, which is to be administered after surgical resection of the carcinoma.

14. The agent for prolonging the recurrence-free survival time according to claim 13, where the surgical resection is complete surgical resection.

15. The agent for prolonging the recurrence-free survival time according to claim 14, which is to be orally administered daily for at least two years after the surgery.

16. An agent for prolonging the recurrence-free survival time of patients in the histopathological stage I of lung squamous cell carcinoma, containing ubenimex or a pharmacologically acceptable salt thereof as the active ingredient for oral administration at 5 to 500 mg/adult • day on the active ingredient basis to the patients after complete surgical resection of lung squamous cell carcinoma in the histopathological stage I.

17. The agent for prolonging the recurrence-free survival time according to claim 16, which is orally administered daily in the ubenimex or a pharmacologically acceptable salt thereof at 10 to 300 mg/adult • day for at least two years after the surgery.
